# EUROPÄISCHE PATENTANMELDUNG

(11) **EP 4 464 238 A1**
(43) Veröffentlichungstag der Anmeldung: **20.11.2024**
(21) Anmeldenummer: 24176024.8
(22) Anmeldetag: 15.05.2024
(51) Int. Cl.: A61B 5/00, A63B 24/00, A61B 5/0205, A61B 5/08

(54) **VERFAHREN ZUM STEUERN EINES SPIROERGOMETRIESYSTEMS**

(30) Priorität: 15.05.2023 DE 102023112800
(71) Anmelder: Technische Universität Dortmund, Körperschaft des Öffentlichen Rechts, 44227 Dortmund (DE)
(72) Erfinder: Wenzel, Charlotte, 44225 Dortmund (DE); Zimmer, Philipp, 50825 Köln (DE)
(74) Vertreter: Michalski Hüttermann & Partner Patentanwälte mbB

(57) **Zusammenfassung**

Die Erfindung betrifft ein Verfahren zum Steuern eines Spiroergometriesystems, wobei das Spiroergometriesystem ein Cardiogerät zur körperlichen Belastung eines Patienten umfasst, aufweisend die folgenden Verfahrensschritte:
a) Erfassen von Patientendaten vor der körperlichen Belastung,
b) Erfassen von Vitalparametern während der körperlichen Belastung,
c) Erfassen von Belastungsparametern während der körperlichen Belastung, umfassend eine Ist-Leistung des Patienten während der körperlichen Belastung,
d) Ermitteln einer optimalen Soll-Leistung des Patienten während der körperlichen Belastung mittels künstlicher Intelligenz abhängig von den Patientendaten, Vitalparametern und Belastungsparametern,
e) Anpassen der Ist-Leistung an die ermittelte Soll-Leistung durch Einstellen des Cardiogeräts,
f) Wiederholen der Schritte b) bis e) in vorbestimmten Zeitabschnitten.

Auf diese Weise wird ein Verfahren zum Steuern eines Spiroergometriesystems bereitgestellt, mit dem die Belastungsleistung während der Messung individuell an die tatsächliche Leistung des Patienten angepasst werden kann.

## Beschreibung

Die Erfindung betrifft ein Verfahren zum Steuern eines Spiroergometriesystems, wobei das Spiroergometriesystem ein Cardiogerät zur körperlichen Belastung eines Patienten umfasst.

Die Spiroergometrie ist ein nicht invasives diagnostisches Verfahren bei dem durch Messung von Atemgasen während körperlicher Belastung die Reaktion von Herz, Kreislauf, Atmung und Stoffwechsel sowie die kardiopulmonale Leistungsfähigkeit eines Patienten qualitativ und quantitativ untersucht wird. Mithilfe der hohen diagnostischen, prognostischen und funktionellen Aussagekraft kann dieses Verfahren in vielen Bereichen angewandt werden. Zu den häufigsten Anwendungsgebieten zählt insbesondere die Messung der Ausdauerleistungsfähigkeit, die Messung des Energiestoffwechsels und die Untersuchung der Leistungsfähigkeit des Atemsystems, sowie die medizinischen Untersuchungen bei erkrankten Patienten.

Die Atemgasmessung erfolgt mit einem Spirometriegerät, das die Exspirationsluft des Patienten analysiert. Die Belastungssteuerung erfolgt dabei mit Hilfe eines Cardiogeräts. Der Patient trägt während der Messung eine Gesichtsmaske, an die ein Volumenstromsensor zur Messung des ventilierten Luftvolumens sowie ein dünner Schlauch, die sogenannte Absaugstrecke, angeschlossen sind. Über die Absaugstrecke wird ein Teil der Exspirationsluft zu den Gassensensoren im Spiroergometriegerät geleitet, wo ihr Gasgehalt analysiert wird. Der prozentuale Gasgehalt der Exspirationsluft wird mit dem der Umgebungsluft verglichen. Zur Berechnung absoluter Werte werden die Differenzen der Gaskonzentrationen mit dem ventilierten Luftvolumen multipliziert. Mit modernen Methoden kann die Gaskonzentration jedes Atemzuges analysiert werden.

Die wichtigsten Atemgasparameter, die bei der Spiroergometrie erfasst werden, sind insbesondere: funktionelle Residualkapazität (FRC), intrathorakales Gasvolumen (ITGV), endexspiratorisches Lungenvolumen (EELV), Ventilation (VE), Atemfrequenz (BF), Atemreserve (BR), Sauerstoffaufnahme (V̇O₂), Kohlendioxidabgabe (V̇CO₂). Daraus lassen sich weitere Parameter bestimmen: Respiratorischer Quotient (RER = V̇CO₂/V̇O₂), Sauerstoffpuls (V̇O₂/Herzfrequenz), Atemäquivalent für O₂ (AÄO2 = V̇E/V̇O₂), Atemäquivalent für CO₂ (AÄCO₂ = VE /V̇CO₂), Atemzugvolumen (VT= V̇E/V̇O₂) und endexspiratorische Partialdruckwerte (PET O₂, PET CO₂).

Herkömmlicherweise werden Spiroergometrien auf dem Laufband oder dem Fahrradergometer mithilfe von Belastungsprotokollen durchgeführt. Diese Protokolle können einen erheblichen Einfluss auf die Belastungsleistung und somit auch auf die Testergebnisse haben. Große und ungleiche Belastungssteigerungen führen zu ungenauen Schätzungen der körperlichen Leistungsfähigkeit, besonders bei kardiovaskulär erkrankten Personen. Zudem führt eine zu schnelle und zu große Belastungssteigerung zur Überschätzung der Belastungsfähigkeit und zu einer geringeren Sensitivität für die Erkennung von Erkrankungen. Ferner kann aus einer zu hohen Startlast und einer inadäquaten Steigerung ein frühzeitiger Testabbruch resultieren, sodass die Ergebnisse unbrauchbar sind. Diese Problematiken stellen besonders im medizinischen Bereich als auch im Breiten- und Leistungssport eine zentrale Limitation dar.

Davon ausgehend ist es die Aufgabe der Erfindung, ein Verfahren zum Steuern eines Spiroergometriesystems bereitzustellen, mit dem die Belastungsleistung während der Messung individuell an die tatsächliche Leistung des Patienten angepasst werden kann.

Diese Aufgabe wird durch den Gegenstand des Patentanspruchs 1 gelöst. Bevorzugte Weiterbildungen finden sich in den Unteransprüchen.

Erfindungsgemäß ist somit ein Verfahren zum Steuern eines Spiroergometriesystems vorgesehen, wobei das Spiroergometriesystem ein Cardiogerät zur körperlichen Belastung eines Patienten umfasst, aufweisend die folgenden Verfahrensschritte:
a) Erfassen von Patientendaten vor der körperlichen Belastung,
b) Erfassen von Vitalparametern während der körperlichen Belastung,
c) Erfassen von Belastungsparametern während der körperlichen Belastung, umfassend eine Ist-Leistung des Patienten während der körperlichen Belastung,
d) Ermitteln einer optimalen Soll-Leistung des Patienten während der körperlichen Belastung mittels künstlicher Intelligenz abhängig von den Patientendaten, Vitalparametern und Belastungsparametern,
e) Anpassen der Ist-Leistung an die ermittelte Soll-Leistung durch Einstellen des Cardiogeräts,
f) Wiederholen der Schritte b) bis e) in vorbestimmten Zeitabschnitten.

Die "optimale Soll-Leistung" wird vorliegend vorzugsweise über die optimale Ausbelastung der Testperson in möglichst kurzer Zeit definiert. Diese Bewertung wird anhand von Ausbelastungskriterien bestimmt. Die Ausbelastungskriterien werden beispielsweise durch die ACSM (engl. American College of Sports Medicine) Empfehlungen definiert. Besonders bevorzugt wird dabei das Ausbelastungskriterium "peak RER" ≥ 1.1 herangezogen, das den Höchstwert des respiratorischen Quotienten angibt.

Es ist somit ein maßgeblicher Punkt der Erfindung, dass die Vitalparameter und die Belastungsparameter in iterativen Schritten gemessen werden und die Belastung des Patienten sowie die Soll-Leistung während der Messung individuell angepasst werden kann, sodass eine optimierte an den Patienten angepasste Belastungskurve erzeugt werden kann. Die Anpassung erfolgt über das Einstellen des Cardiogeräts. Dies kann insbesondere das Erhöhen oder Verringern eines Widerstands oder einer Geschwindigkeit umfassen.

Das Verfahren bringt den Vorteil mit sich, dass der Belastungstest frühzeitig abgebrochen werden kann und somit auch Patienten teilnehmen können, die körperlich eingeschränkt oder erkrankt sind und gar keine volle Ausbelastung erreichen könnten. Da durch die stetigen Vorhersagen der Soll-Leistung mithilfe der KI-Modelle und die entsprechende Anpassung des Protokolls, wie beispielsweise die Anpassung des Widerstandes auf den Radergometer, eine immer präziser werdende Soll-Leistung vorhergesagt werden kann, wäre ab dem Zeitpunkt, wo die Soll-Leistung möglichst akkurat vorhergesagt wird eine Testbeendigung sinnvoll. Anschließend kann mit der Soll-Leistung beispielsweise weiteres Training gesteuert werden.

Gemäß einer bevorzugten Weiterbildung der Erfindung umfasst das Verfahren folgende weitere Verfahrensschritte:
aa) Ermitteln einer Start-Soll-Leistung des Patienten vor der körperlichen Belastung mittels künstlicher Intelligenz abhängig von den Patientendaten,
ab) Einstellen des Cardiogeräts entsprechend der ermittelten Start-Soll-Leistung für den Start der körperlichen Belastung.

Bereits vor der körperlichen Belastung, nachdem Patientendaten erfasst wurden, kann abhängig von den Patientendaten eine Start-Soll-Leistung ermittelt werden, um einen ersten Anhaltspunkt für die anschließende Anpassung der Belastungskurve während der körperlichen Belastung zu haben. Das Cardiogerät wird dann entsprechend der ermittelten Start-Soll-Leistung voreingestellt, bevor die körperliche Belastung beginnt. Dadurch wird gewährleistet, dass eine erste Annäherung an die individuell angepasste Belastungskurve erfolgen kann, bevor die körperliche Belastung beginnt.

Gemäß einer bevorzugten Weiterbildung der Erfindung sind die vorbestimmten Zeitabschnitte derart klein gewählt, dass eine kontinuierliche Überwachung und Anpassung erfolgt. Mit "kontinuierlich" ist vorliegend gemeint, dass die Erhebung der Daten bzw. die Messung der Belastung laufend während der Ausbelastung in mehreren zeitlich aneinander angrenzenden Intervallen erfolgt. Die Anpassung der Soll-Leistung erfolgt dann in regelmäßigen Zeitabschnitten nach der Auswertung der erhobenen Daten eines Messintervalls. Sobald ein Messintervall abgeschlossen ist, wird direkt das nächste gestartet und ausgewertet. Dadurch entsteht eine individuelle Belastungskurve mit einer hohen Auflösung und Genauigkeit, die durch die Länge des Messintervalls bestimmt wird.

Gemäß einer bevorzugten Weiterbildung der Erfindung umfassen die Patientendaten folgende Daten: Demografische Daten, wie insbesondere biologisches Geschlecht, Alter, höchster Bildungsabschluss, Beruf, Verwendung von Verhütungsmitteln, chronische Vorerkrankungen, Allergien, regelmäßige Medikamenteneinnahme, regelmäßige Einnahme von Nahrungsergänzungsmitteln, Alkoholkonsum, Rauchverhalten, Drogenkonsum; anthropometrische Daten, wie insbesondere Größe, Gewicht, Hüftumfang, Taillenumfang und sich daraus ergeben Parameter wie der body-mass-index (BMI) oder die Waist-Hip-Ratio; die Körperzusammensetzung, wie insbesondere Fettmasse, Körperzellmasse, extrazelluläre Masse, extrazelluläres Wasser, intrazelluläres Wasser und sich daraus ergebene Parameter wie beispielsweise das viszerale Fett oder die Skelettmuskelmasse; weitere Daten, wie insbesondere Handkraft, körperliches Aktivitätsniveau, Chronotyp, Biomarker aus Speichelproben, Schlafqualität, Blutparameter, wie insbesondere WBC, RBC, HGB, HCT, MCV, MCH, MCHC, PLT, LYM%, MXD%, NEUT%, LYM#, MXD#, NEUT#, RDW-SD, RDW-CV, PDW, MPV, P-LCR, PCT. Es hat sich gezeigt, dass sich die Speichelproben sehr gut für eine präzise Vorhersagen eignen. Vor jeder Testung nehmen wir diese und anschließend werden höchst Aufwendige Biomarkeranalysen betrieben (z.B. Proteomik).

Gemäß einer weiteren bevorzugten Weiterbildung der Erfindung umfassen die Vitalparameter folgende Daten: Herzfrequenz (HF), Blutdruck, Atemgaszusammensetzung, funktionelle Residualkapazität (FRC), intrathorakales Gasvolumen (ITGV), endexspiratorisches Lungenvolumen (EELV), Ventilation (VE), Atemfrequenz (BF), Atemreserve (BR), Sauerstoffaufnahme (V̇O₂), Kohlendioxidabgabe (V̇CO₂), ventilatorische Schwellenwerte (VT₁, VT₂) und/oder maximale Sauerstoffaufnahme (V̇O₂ₘₐₓ). Daraus lassen sich weitere Parameter bestimmen, wie insbesondere der respiratorische Quotient (RER = V̇CO₂/V̇O₂), Sauerstoffpuls (V̇O₂/Herzfrequenz), Atemäquivalent für O₂ (AÄO2 = VE / V̇O₂), Atemäquivalent für CO₂ (AÄCO₂ = VE / V̇CO₂), Atemzugvolumen (VT= V̇E/ V̇O₂) und/oder endexspiratorische Partialdruckwerte (PET O₂, PET CO₂).

Gemäß einer bevorzugten Weiterbildung der Erfindung umfassen die Belastungsparameter zusätzlich zur Ist-Leistung folgende Daten: die Zeit und/oder Borg-Werte. Die Borg-Werte werden zu verschiedenen Zeitpunkten erhoben. Diese werden mittels einer Borg-Skala erfasst, welche ein Hilfsmittel zur Bewertung des subjektiven Belastungsempfinden ist. Zum Messen der Borg-Werte zeigt der Patient während der Belastung auf die Borg-Skala.

Gemäß einer bevorzugten Weiterbildung der Erfindung wird die künstliche Intelligenz durch die Methode des überwachten Lernens angelernt. Der Algorithmus lernt eine Funktion aus gegebenen Paaren von Ein- und Ausgaben. Dabei stellt während des Lernens ein "Lehrer" den korrekten Funktionswert zu einer Eingabe bereit. Ziel beim überwachten Lernen ist, dass dem Netz nach mehreren Rechengängen mit unterschiedlichen Ein- und Ausgaben die Fähigkeit antrainiert wird, Assoziationen herzustellen. Dabei sind sowohl die Eingabedaten wie auch die korrekten Zielvariablen im Datensatz enthalten. Durch das Bereitstellen von Trainings- und Testdaten, kann der Algorithmus komplexe Muster und Zusammenhänge erkennen und somit aus den Daten lernen. Diesen Prozess nennt man auch Modelltraining. Trainiert wird die künstliche Intelligenz anhand eines Testdatensatzes. Der vorzugsweise laufend wachsende Testdatensatz umfasst eine Vielzahl von Patientendaten, Vitalparametern und Belastungsparametern von vorab durchgeführten Testmessungen unterschiedlicher Patienten aus unterschiedlichen Bevölkerungsgruppen. Die Patienten des Testdatensatzes erreichten eine optimale Ausbelastung, die besonders bevorzugt durch das Ausbelastungskriterium "peak RER" ≥ 1.1 definiert ist. Die Patienten dieses Testdatensatzes erreichten eine Ausbelastung nach unterschiedlichen Zeitpunkten. Um schließlich mit unbekannten Patienten eine optimale Ausbelastung in möglichst kurzer Zeit zu erreichen, wird eine KI mit den bereits existierenden Testdatensatz so trainiert, dass die optimale Ausbelastung berechnet bzw. vorhergesagt und das Belastungsprotokoll entsprechend angepasst wird, sodass in möglichst kurzer Zeit die errechnete optimale Ausbelastung erreicht wird. Unter "möglichst kurzer Zeit" wird insbesondere die minimal notwendige Zeit verstanden, in der eine optimale Ausbelastung möglich ist. Dieses Vorgehen wird zu jedem Anpassungszeitpunkt durchlaufen, sodass eine individuelles Belastungsprotokoll entsteht. Alternativ kann gemäß einer bevorzugten Weiterbildung der Erfindung die künstliche Intelligenz durch die Methode des nicht überwachten Lernens und/oder Deeplearning angelernt werden.

Gemäß einer bevorzugten Weiterbildung der Erfindung wird die künstliche Intelligenz mittels eines Algorithmus angelernt, wobei der Algorithmus aus folgender Liste wählbar ist: Künstliches neuronales Netz, Support Vector Machine, Reinforcement, Entscheidungsbaum, Lineare Regression, Nächste-Nachbar-Klassifikation, Zeitreihenmodelle, XGBoost, Gradient Boosting und/oder Random Forest.

Das künstliche neuronale Netz ist Aufbau des menschlichen Nervensystems nachempfunden. Durch Training werden bestimmte Kanäle stärker frequentiert und ausgebaut. Künstliche neuronale Netzwerke sind aus Knoten, den sogenannten Neuronen, aufgebaut. Dabei werden drei Arten von Neuronen unterschieden: Input Neuronen, die Information aufnehmen und diese weiterleiten. Sie befinden sich in der Eingabeschicht; Hidden Neuronen, die Reize aufnehmen und diese weiterleiten. Sie befinden sich im sogenannten hidden layer; und Output Neuronen, die Reize aufnehmen und verarbeitete Informationen ausgeben. Diese Neuronen befinden sich in der Ausgabeschicht.

Eine Support Vector Machine ist ein Klassifikator, der eine Menge von Objekten so in Klassen unterteilt, dass um die Klassengrenzen herum ein möglichst breiter Bereich frei von Objekten bleibt. Support Vector Machines können sowohl zur Klassifizierung als auch zur Regression verwendet werden.

Bei dem Reinforcement Learning beobachtet ein sogenannter "Agent" die Umgebung und wählt eine angemessene Aktion. Anschließend schickt die Umgebung ein Feedbacksignal, das die Aktion mit einem Wert versieht. Dieser Vorgang wird als "Belohnung" betitelt, sodass der Agent erkennt, ob die Entscheidung gut war und es sich lohnt, sie in ähnlichen Situationen zu wiederholen. War es eine schlechte Entscheidung mit einem unerwünschten Ergebnis, gilt es diese weiter zu vermeiden.

Bei einem Entscheidungsbaum handelt es sich insbesondere um geordnete, gerichtete Bäume, die der Darstellung von Entscheidungsregeln dienen. Die grafische Darstellung als Baumdiagramm veranschaulicht hierarchisch aufeinanderfolgende Entscheidungen. Sie dienen zur automatischen Klassifikation oder zur Herleitung von formalen Regeln aus Erfahrungswissen.

Die lineare Regression wird insbesondere als ein Spezialfall der Regressionsanalyse, also als ein statistisches Verfahren, mit dem versucht wird, eine beobachtete abhängige Variable durch eine oder mehrere unabhängige Variablen zu erklären, verstanden. Bei der linearen Regression wird dabei ein lineares Modell angenommen. Es werden also nur solche Zusammenhänge herangezogen, bei denen die abhängige Variable eine Linearkombination der Regressionskoeffizienten ist.

Die Nächste-Nachbarn-Klassifikation umfasst insbesondere eine nichtparametrische Methode zur Schätzung von Wahrscheinlichkeitsdichtefunktionen. Der daraus resultierende K-Nearest-Neighbor-Algorithmus (KNN, zu Deutsch "k-nächste-Nachbarn-Algorithmus") ist ein Klassifikationsverfahren, bei dem eine Klassenzuordnung unter Berücksichtigung seiner k nächsten Nachbarn vorgenommen wird. Der Teil des Lernens besteht aus simplem Abspeichern der Trainingsbeispiele, was auch vorzugsweise als lazy learning ("träges Lernen") bezeichnet wird.

Der Random-Forest Algorithmus umfasst insbesondere ein Klassifikations- und Regressionsverfahren, das aus mehreren unkorrelierten Entscheidungsbäumen besteht. Alle Entscheidungsbäume sind unter einer bestimmten Art von Randomisierung während des Lernprozesses gewachsen. Für eine Klassifikation darf jeder Baum in diesem Wald eine Entscheidung treffen und die Klasse mit den meisten Stimmen entscheidet die endgültige Klassifikation. Random Forests können auch zur Regression eingesetzt werden.

Vorzugsweise ist eine im Anlernprozess der künstlichen Intelligenz eine Kreuzvalidierung vorgesehen, sodass das erlernte Muster auf unbekannten Daten anwendbar ist.

Erfindungsgemäß ist weiterhin ein Spiroergometriesystem zum Messen von Atemgasen eines Patienten während einer körperlichen Belastung vorgesehen. Das Spiroergometriesystem umfasst
ein Cardiogerät zur körperlichen Belastung des Patienten,
eine erste Messeinheit zum Erfassen von Vitalparametern,
eine zweite Messeinheit zum Erfassen von Belastungsparametern,
eine Auswerteeinheit zum Auswerten der erfassten Vitalparameter und Belastungsparameter und eine Steuereinheit zum Steuern des Cardiogeräts, wobei die Auswerteeinheit und die Steuereinheit zum Durchführen der Verfahrensschritte nach einem der vorherigen Ansprüche ausgestaltet sind.

Die Atemgasmessung erfolgt folglich mit einem Spiroergometriesystem. Eine erste Messeinheit, umfassend vorzugsweise ein Atemgasmessgerät, ein Blutdruckmessgerät und/oder ein Herzfrequenzmessgerät, misst die Vitalparameter, insbesondere den Blutdruck sowie die Herzfrequenz und analysieren die Exspirationsluft (Ausatemluft) des Patienten. Die Belastungssteuerung erfolgt dabei mit Hilfe eines Cardiogeräts, insbesondere ein Laufband oder Fahrradergometer. Der Patient trägt während der Messung eine Gesichtsmaske, an die ein Volumenstromsensor zur Messung des ventilierten Luftvolumens sowie ein dünner Schlauch, die sogenannte Absaugstrecke, angeschlossen sind. Über die Absaugstrecke wird ein Teil der Exspirationsluft zu Gassensensoren im Atemgasmessgerät geleitet, wo ihr Gasgehalt analysiert wird. Der prozentuale Gasgehalt der Exspirationsluft wird mit dem der Umgebungsluft verglichen. Zur Berechnung absoluter Werte werden die Differenzen der Gaskonzentrationen mit dem ventilierten Luftvolumen multipliziert.

Die zweite Messeinheit umfasst einen Widerstands- und/oder einen Geschwindigkeitssensor, mit denen die auf dem Cardiogerät durch den Patienten vollbrachte Leistung gemessen werden kann. Dabei handelt es sich um die Ist-Leistung. Die Ist-Leistung wird definiert über die von dem Patienten aufgebrachte Energie bzw. verrichteter Arbeit pro Zeitabschnitt. Bei der Soll-Leistung handelt es sich um die ermittelte Leistung, die der Patient verrichten sollte, damit die Belastung abhängig von den ermittelten Patientendaten, Vitalparameter und Belastungsparameter optimal ausgelegt ist und eine optimale Messung stattfinden kann. Diese Soll-Leistung wird von der Auswerteeinheit unter Berücksichtigung aller ermittelten Parameter bestimmt. Mittels der Steuereinheit wird das Cardiogerät entsprechend angepasst, indem beispielsweise beim Laufband die Geschwindigkeit und/oder die Neigung erhöht oder verringert bzw. beim Ergometer der Widerstand erhöht oder verringert wird, sodass die Ist-Leistung des Patienten der Soll-Leistung angenähert wird.

Erfindungsgemäß ist weiterhin ein Computerprogrammprodukt vorgesehen, umfassend Anweisungen, die wenn sie auf einem Prozessor ausgeführt werden, ein oben beschriebenes Spiroergometriesystem veranlassen, ein oben beschriebenes Verfahren durchzuführen.

Nachfolgend wird die Erfindung anhand eines bevorzugten Ausführungsbeispiels unter Bezugnahme auf die Zeichnungen weiter im Detail erläutert.

In den Zeichnungen zeigen
- Fig. 1a, b: schematisch Belastungsprotokolle eines Patienten gemäß dem Stand der Technik,
- Fig. 2: schematisch ein Belastungsprotokoll eines Patienten gemäß einer bevorzugten Ausgestaltung der Erfindung,
- Fig. 3: schematisch ein Verfahren zum Steuern eines Spiroergometriesystems gemäß einer bevorzugten Ausgestaltung der Erfindung.

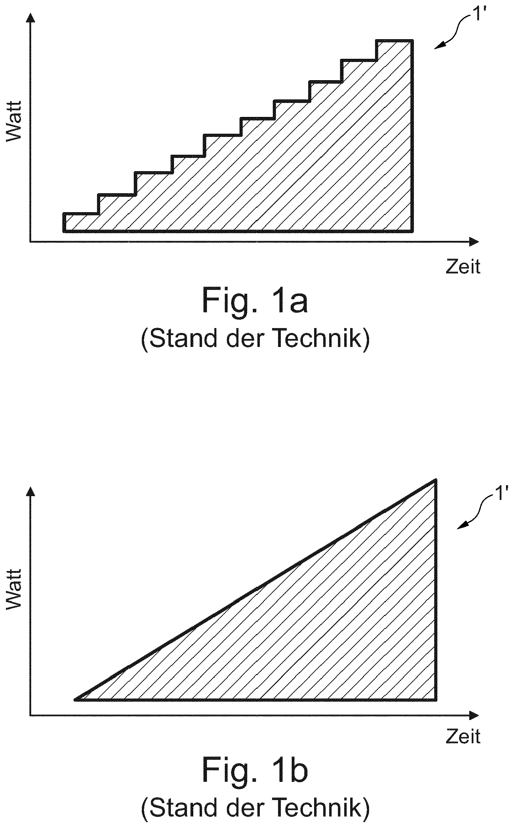

Fig. 1a und Fig. 1b zeigen jeweils ein Belastungsprotokoll 1` gemäß dem Stand der Technik. Dabei zeigt die Fig. 1a ein sogenanntes Stufenprotokoll und die Fig. 1b ein sogenanntes Rampenprotokoll. Diese vordefinierten Belastungsprotokolle 1` werden vorab abhängig von den Patientendaten und der daraus abgeleiteten zu erwartenden Leistungsfähigkeit des Patienten ausgewählt. Die Wahl des Belastungsprotokolls kann einen erheblichen Einfluss auf die Belastungsleistung und somit auf die Messwerte und das Testergebnis haben. Ungeeignet große oder ungleiche Belastungssteigerungen führen zu ungenauen Schätzungen der körperlichen Leistungsfähigkeit. Dies ist insbesondere bei kardiovaskulär erkrankten Patienten der Fall. Eine zu schnelle und zu große Belastungssteigerung führt zur Überschätzung der Belastungsfähigkeit und zu einer geringeren Sensitivität für die Erkennung von Erkrankungen. Eine zu hohe Startlast oder eine inadäquate Steigerung kann zu einem frühzeitigen Testabbruch führen, sodass die Messungen unbrauchbar sind.

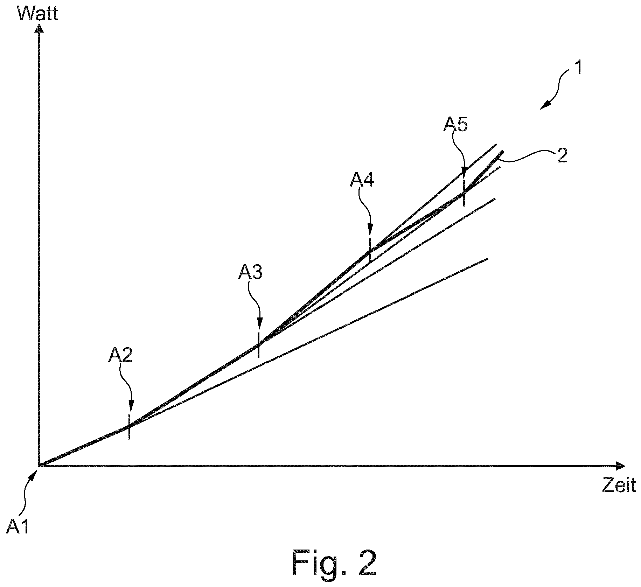

Fig. 2 zeigt ein individuell an den Patienten angepasstes Belastungsprotokoll 1. Die Belastung des Patienten bzw. die Leistung wird während der Messung, also im Zeitverlauf, nicht nach einem vordefinierten Schema erhöht. Die Steigung der Belastungskurve 2 ist nicht stetig oder folgt keinem bestimmten Muster. Die für die Messung optimale Leistung des Patienten wird für jeden Zeitschritt bzw. nach jedem Messintervall neu bestimmt und individuell angepasst. Dabei werden die Vitalparameter und Belastungsparameter laufend gemessen und fließen zusammen mit den Patientendaten instantan in die Bestimmung der Soll-Leistung ein. Zur Ermittlung der optimalen Soll-Leistung wird eine künstliche Intelligenz verwendet, die dazu trainiert ist, ausgehend von den Patientendaten, Vitalparametern und Belastungsparametern die optimale Soll-Leistung zu bestimmen. Somit kann die optimale Leistung bestimmt und durch Anpassen des Cardiogeräts direkt umgesetzt werden. Zu Beginn, also vor der körperlichen Belastung zum Zeitpunkt t = 0 und nach jedem Messintervall wird die Belastungskurve angepasst. Dies wird in der Fig. 2 durch die gezeigten Anpassungspunkte A1-A5 verdeutlicht. An den Anpassungspunkte A1-A5 ändert sich der Verlauf der Belastungskurve 1 im Vergleich zu den ebenfalls dargestellten stetigen Verläufen. Es entsteht "live" während der Messung eine an den Patienten individuell angepasste Belastungskurve 2 und ein für den individuellen Patienten optimiertes Belastungsprotokoll 1.

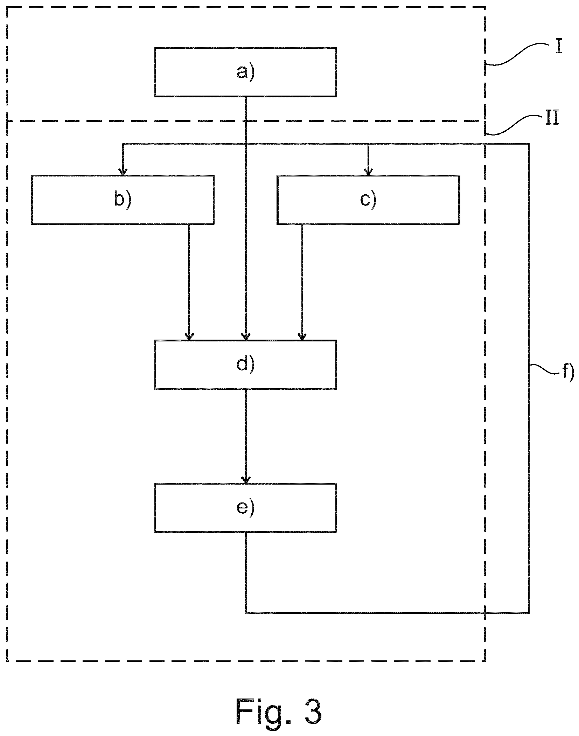

Fig. 3 zeigt ein schematisches Flussdiagramm für ein Verfahren zum Steuern eines Spiroergometriesystems gemäß einem bevorzugten Ausführungsbeispiel der Erfindung. Vor der körperlichen Belastung des Patienten I werden die Patientendaten erfasst a. Dazu zählen insbesondere die Größe, das Gewicht, das Alter, das Geschlecht und die Körperzusammensetzung des Patienten. Danach wird die körperliche Belastung gestartet, indem der Patient damit beginnt, das Cardiogerät zu betätigen, beispielsweise auf einem Laufband zu laufen oder auf einem Fahrradergometer zu treten. Während der körperlichen Belastung II werden die Vitalparameter und die Belastungsparameter laufend gemessen b, c. Zu den Vitalparametern gehören insbesondere neben der Herzfrequenz und dem Blutdruck auch die Daten aus der Atemgasanalyse. Die Belastungsparameter geben einen Aufschluss über die Ist-Leistung des Patienten, also über die Leistung, die der Patient während der körperlichen Belastung erbringt. Ausgehend von den Patientendaten, den Vitalparametern und den Belastungsparametern wird mittels künstlicher Intelligenz eine optimale Soll-Leistung bestimmt d. Das Cardiogerät wird anschließend derart angepasst, dass die Ist-Leistung an die Soll-Leistung wenigstens angenähert wird e. Anschließend werden die Schritte b bis e wiederholt, sodass eine kontinuierliche Messung und Anpassung des Cardiogeräts gewährleistet werden kann. Zustandsänderungen können direkt erkannt und berücksichtigt werden, sodass eine individuell an den Patienten angepasst Belastungskurve und somit ein optimiertes Belastungsprotokoll entsteht.

### Bezugszeichenliste

- 1': Belastungsprotokoll gemäß dem Stand der Technik
- 1: Individuelles Belastungsprotokoll
- 2: Belastungskurve

- A1-A5: Anpassungspunkte

- a): Erfassen von Patientendaten vor der körperlichen Belastung
- b): Erfassen von Vitalparametern während der körperlichen Belastung
- c): Erfassen von Belastungsparametern während der körperlichen Belastung
- d): Ermitteln einer optimalen Soll-Leistung des Patienten während der körperlichen Belastung
- e): Anpassen der Ist-Leistung an die ermittelte Soll-Leistung
- f): Wiederholen der Schritte b) bis e) in vorbestimmten Zeitabschnitten

- I: Verfahren vor der körperlichen Belastung
- II: Verfahren während der körperlichen Belastung

## Patentansprüche

1. Verfahren zum Steuern eines Spiroergometriesystems, wobei das Spiroergometriesystem ein Cardiogerät zur körperlichen Belastung eines Patienten umfasst, aufweisend die folgenden Verfahrensschritte:
a) Erfassen von Patientendaten vor der körperlichen Belastung,
b) Erfassen von Vitalparametern während der körperlichen Belastung,
c) Erfassen von Belastungsparametern während der körperlichen Belastung, umfassend eine Ist-Leistung des Patienten während der körperlichen Belastung,
d) Ermitteln einer optimalen Soll-Leistung des Patienten während der körperlichen Belastung mittels künstlicher Intelligenz abhängig von den Patientendaten, Vitalparametern und Belastungsparametern,
e) Anpassen der Ist-Leistung an die ermittelte Soll-Leistung durch Einstellen des Cardiogeräts,
f) Wiederholen der Schritte b) bis e) in vorbestimmten Zeitabschnitten.

2. Verfahren nach Anspruch 1, mit folgenden weiteren Verfahrensschritten:
aa) Ermitteln einer Start-Soll-Leistung des Patienten vor der körperlichen Belastung mittels künstlicher Intelligenz abhängig von den Patientendaten,
ab) Einstellen des Cardiogeräts entsprechend der ermittelten Start-Soll-Leistung für den Start der körperlichen Belastung.

3. Verfahren nach Anspruch 1 oder 2, wobei die vorbestimmten Zeitabschnitte derart klein gewählt sind, dass eine kontinuierliche Überwachung und Anpassung erfolgt.

4. Verfahren nach einem der vorherigen Ansprüche, wobei die Patientendaten folgende Daten umfassen: Demografische Daten, anthropometrische Daten, Daten betreffend die Körperzusammensetzung, Handkraft, körperliches Aktivitätsniveau, Schlafqualität und/oder Blutparameter.

5. Verfahren nach einem der vorherigen Ansprüche, wobei die Vitalparameter folgende Daten umfassen: Herzfrequenz, Blutdruck, Atemgaszusammensetzung, funktionelle Residualkapazität, intrathorakales Gasvolumen, endexspiratorisches Lungenvolumen, Ventilation, Atemfrequenz, Atemreserve, Sauerstoffaufnahme, Kohlendioxidabgabe, ventilatorische Schwellenwerte, maximale Sauerstoffaufnahme, der respiratorische Quotient, Sauerstoffpuls, Atemäquivalent für O₂, Atemäquivalent für CO₂, Atemzugvolumen und/oder endexspiratorische Partialdruckwerte.

6. Verfahren nach einem der vorherigen Ansprüche, wobei die Belastungsparameter zusätzlich zur Ist-Leistung folgende Daten umfassen: die Zeit und/oder Borg-Werte.

7. Verfahren nach einem der vorherigen Ansprüche, wobei die künstliche Intelligenz durch die Methode des überwachten Lernens angelernt wird.

8. Verfahren nach einem der vorherigen Ansprüche, wobei die künstliche Intelligenz mittels eines Algorithmus angelernt wird, wobei der Algorithmus aus folgender Liste wählbar ist: Künstliche neuronales Netz, Support Vector Machine, Reinforcement, Entscheidungsbaum, Lineare Regression, Nächste-Nachbar-Klassifikation und/oder Random Forest.

9. Spiroergometriesystem zum Messen von Atemgasen eines Patienten während einer körperlichen Belastung, umfassend
ein Cardiogerät zur körperlichen Belastung des Patienten,
eine erste Messeinheit zum Erfassen von Vitalparametern,
eine zweite Messeinheit zum Erfassen von Belastungsparametern,
eine Auswerteeinheit zum Auswerten der erfassten Vitalparameter und Belastungsparameter und eine Steuereinheit zum Steuern des Cardiogeräts, wobei die Auswerteeinheit und die Steuereinheit zum Durchführen der Verfahrensschritte nach einem der vorherigen Ansprüche ausgestaltet sind.

10. Computerprogrammprodukt, umfassend Anweisungen, die wenn sie auf einem Prozessor ausgeführt werden, ein Spiroergometriesystem nach Anspruch 9 veranlassen, ein Verfahren nach einem der Ansprüche 1 bis 8 durchzuführen.
